(19) Europäisches Patentamt
European Patent Office
Office européen des brevets

(11) Veröffentlichungsnummer: **0 094 351**
**B1**

(12) # EUROPÄISCHE PATENTSCHRIFT

(45) Veröffentlichungstag der Patentschrift: **23.07.86**

(51) Int. Cl.⁴: **C 07 D 309/40**, A 01 N 25/32

(21) Anmeldenummer: **83810190.5**

(22) Anmeldetag: **04.05.83**

(54) **Cyclopropancarbonsäurederivate.**

(30) Priorität: **10.05.82 CH 2891/82**

(43) Veröffentlichungstag der Anmeldung: **16.11.83 Patentblatt 83/46**

(45) Bekanntmachung des Hinweises auf die Patenterteilung: **23.07.86 Patentblatt 86/30**

(84) Benannte Vertragsstaaten: **AT BE CH DE FR GB IT LI NL**

(56) Entgegenhaltungen: **EP-A-0 041 021**

(73) Patentinhaber: **CIBA- GEIGY AG, Klybeckstrasse 141, CH- 4002 Basel (CH)**

(72) Erfinder: **Gsell, Laurenz, Dr., Maiengasse 56, CH- 4056 Basel (CH)**

**EP 0 094 351 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Cyclopropancarbonsäurederivate, Verfahren zu ihrer Herstellung, Mittel, welche die Cyclopropancarbonsäurederivate als Wirkstoffe enthalten, und ihre Verwendung zum Schützen von Kulturpflanzen gegen schädigende Wirkungen von Agrarchemikalien.

Beim Einsatz von Agrarchemikalien, wie Pflanzenschutzmitteln und insbesondere Herbiziden, können in Abhängigkeit von Faktoren wie beispielsweise Dosis der Agrarchemikalie und Applikationsart, Art oder Sorte der Kulturpflanze, Bodenbeschaffenheit und klimatischen Bedingungen, wie beispielsweise Belichtungsdauer, Temperatur und Niederschlagsmengen, die Kulturpflanzen in gewissem Masse geschädigt werden. So ist beispielsweise bekannt, dass Herbizide aus den verschiedensten Stoffklassen, wie Triazine, Harnstoffderivate, Carbamate, Thiolcarbamate, Halogenacetanilide, Halogenphenoxyessigsäuren und anderen Klassen bei der Anwendung in wirksamer Dosis die Kulturpflanzen, welche gegen die nachteilige Wirkung von unerwünschtem Pflanzenwachstum geschützt werden sollen, in gewissem Masse schädigen können. Um diesem Problem zu begegnen, sind schon verschiedene Stoffe vorgeschlagen worden, welche befähigt sind, die schädigende Wirkung eines Herbizids auf die Kulturpflanze spezifisch zu antagonisieren, d.h., die Kulturpflanze zu schützen, ohne zugleich die Herbizidwirkung gegen zu bekämpfende Unkräuter merklich zu beeinflussen. Dabei hat sich jedoch gezeigt, dass die vorgeschlagenen Gegenmittel häufig nur ein enges Einsatzgebiet haben, d.h., ein bestimmtes Gegenmittel eignet sich oftmals nur zur Anwendung bei einzelnen Kulturpflanzenarten und/oder zum Schutz der Kulturpflanzen gegen einzelne herbizide Substanzen oder Stoffklassen.

So beschreibt die GB—A—1,277,557 die Behandlung von Samen bzw. Sprösslingen von Weizen und Sorghum mit gewissen Oxamsäureestern und Amiden zum Schutz vor dem Angriff durch "Alachlor" (N-Methoxymethyl-N-chloracetyl-2,6-diäthylanilin). In DE—A—1,952,910 und DE—A—2,245,471, sowie in FR—B—2,021,611 werden Gegenmittel zur Behandlung von Getreide-, Mais- und Reissamen zum Schutz gegen die schädigende Einwirkung von herbizid wirksamen Thiolcarbamaten vorgeschlagen. Gemäss DE—B—1,567,075 und US—A—3,131,509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in DE—A—2,141,586 und DE—A—2,218,097, sowie in US—A—3,867,444 beschrieben.

Ferner können Maispflanzen gemäss der DE—A—2,402,983 wirksam vor Schädigung durch Chloracetanilide geschützt werden, indem man dem Boden als Gegenmittel ein N-disubstituiertes Dichloracetamid zuführt.

Gemäss EP—A—11.047 können auch Alkoximinobenzylcyanide, deren Alkoxygruppe u.a. durch eine acetalisierte Carbonylgruppe substituiert ist, als Mittel zum Schutz von Kulturpflanzen vor der schädigenden Wirkung von Herbiziden verschiedener Stoffklassen verwendet werden.

Carbalkoxyvinyl-cyclopropancarbonsäure-4-pyron-3-yl-ester mit insektizider Wirkung sind aus der EP—A—41.021 bekannt.

Es wurde nun gefunden, dass sich überraschenderweise eine Gruppe von neuen Cyclopropancarbonsäurederivaten hervorragend dazu eignet, Kulturpflanzen gegen schädigende Wirkungen von Agrarchemikalien, wie beispielsweise Pflanzenschutzmitteln, insbesondere Herbiziden, zu schützen. Diese Cyclopropancarbonsäurederivate werden daher im folgenden auch als "Gegenmittel", "Antidot" oder "Safener" beziechnet.

Die neuen Cyclopropancarbonsäurederivate entsprechen der Formel I

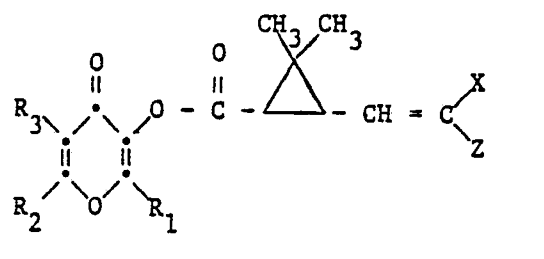

(I),

worin $R_1$, $R_2$ und $R_3$ unabhängig voneinander Wasserstoff; Halogen; $C_1$—$C_8$-Alkyl, welches unsubstituiert oder durch einen oder mehrere Substituenten aus der Gruppe Thiocyan, Hydroxy, Halogen, $C_1$—$C_4$-Alkoxy, $C_1$—$C_4$-Alkylthio, Acyloxy oder $R_4$ substituiert ist; $C_3$—$C_6$-Alkenyl, welches unsubstituiert oder durch $R_5$ substituiert ist; $C_3$—$C_6$-Alkinyl, welches unsubstituiert oder durch $R_6$ substituiert ist; oder $R_7$ bedeuten, wobei $R_4$, $R_5$, $R_6$ und $R_7$ für Phenyl, welches unsubstituiert oder durch maximal 3 gleiche oder verschiedene Substituenten aus der Gruppe Halogen, Nitro, Cyan, $C_{1-4}$-Carbonsäure, $C_{1-4}$-Carbonsäure-$C_{1-8}$-alkylester, $C_{1-8}$-Alkyl, $C_{3-6}$-Alkenyl, $C_{3-6}$-Alkinyl, Halogen-$C_{1-8}$-alkl, $C_{1-8}$-alkyl, $C_{1-8}$-Alkoxy oder $C_{1-8}$-Alkylthio substituiert ist; und X und Z Fluor, Chlor, Brom oder Trifluormethyl bedeuten.

Die Verbindungen der Formel I liegen als Gemisch von verschiedenen optisch aktiven Isomeren vor, wenn bei der Herstellung nicht einheitlich optisch aktive Ausgangsmaterialien verwendet werden. Unter einer Verbindung der Formel I versteht man sowohl die einzelnen optischen und geometrischen Isomere

als auch deren Gemische. Die verschiedenen Isomerengemische können nach bekannten Methoden in die einzelnen Isomeren aufgetrennt werden.

Unter Halogen als Substituent oder Teil eines Substituenten sind Fluor, Chlor, Brom und Jod zu verstehen.

Unter Alkyl als Substituent oder Teil eines Substituenten sind im Rahmen der jeweils angegebenen Anzahl von Kohlenstoffatomen alle in Betracht kommenden Isomere zu verstehen, wie beispielsweise Methyl, Aethyl, n-Propyl, Isopropyl, n-Butyl, sek.-Butyl, tert.-Butyl oder Isobutyl sowie Pentyl, Hexyl, Heptyl und Octyl und ihre Isomere.

Unter Alkyl als Bestandteil der Substituenten $R_4$, $R_5$, $R_6$ und $R_7$ sind vorzugsweise Alkylgruppen mit 1 bis 8, insbesondere 1 bis 4 Kohlenstoffatomen, zu verstehen.

Unter Acyloxy ist vor allem ein gegebenenfalls substituierter, acyclischer Kohlenwasserstoffrest mit maximal 6 Kohlenstoffatomen zu verstehen. Bevorzugt sind Alkylcarbonyloxygruppen mit 1 bis 4 Kohlenstoffatomen, insbesondere Acetoxy.

Unter Alkenyl als Bestandteil der Substituenten $R_4$, $R_6$, $R_6$ und $R_7$ sind vor allem Alkenylgruppen mit 3 bis 6 Kohlenstoffatomen zu verstehen, wie beispielsweise Allyl und Methallyl.

Unter Alkinyl als Bestandteil der Substituenten $R_4$, $R_5$, $R_6$ und $R_7$ sind insbesondere Alkinylgruppen mit 3 bis 6 Kohlenstoffatomen zu verstehen, wie beispielsweise Propargyl und Butinyl-(2).

Die Gruppen Carbonsäure und Carbonsäurealkylester als substituenten von $R_4$, $R_5$, $R_6$ und $R_7$ bedeuten $C_{1-4}$-Carbonsäuren und $C_{1-4}$-Carbonsäurealkylester.

Von den Verbindungen der Formel I sind diejenigen bevorzugt, die einer der nachstehend aufgeführten Gruppen angehören:

a) Verbindungen der Formel I, in denen $R_1$, $R_2$ und $R_3$ die für Formel I angegebenen Bedeutungen haben und X und Z für Chlor stehen;

b) Verbindungen der Formel I, in denen $R_2$ die für Formel I angegebenen Bedeutungen hat, $R_1$ und $R_3$ Wasserstoff und X und Z Chlor bedeuten;

c) Verbindungen der Formel I, in denen $R_1$, X und Z die für Formel I angegebenen Bedeutungen haben und $R_2$ und $R_3$ für Wasserstoff stehen; und

d) Verbindungen der Formel I, in denen $R_1$ $C_1$—$C_4$-Alkyl und $R_2$ und $R_3$ Wasserstoff bedeuten und X und Z die für Formel I angegebenen Bedeutungen haben.

Die Gruppen a), b), c) und d) umfassen die einzelnen Isomere der entsprechenden Verbindungen wie auch Isomerengemische. Besonders erwähnenswerte Verbinungen sind:

2 - (2,2 - Dichlorvinyl) - 3,3 - dimethyl - cyclopropancabonsäure - (2 - methyl - 4 - pyron - 3 - yl) - ester und

2 - (2,2 - Dichlorvinyl) - 3,3 - dimethyl - cyclopropancarbonsäure - (2 - äthyl - 4 - pyron - 3 - yl) - ester.

Die Cyclopropancarbonsäurederivate der Formel I besitzen in hervorragendem Masse die Eigenschaft, Kulturpflanzen gegen schädigende Wirkungen von Agrarchemikalien zu schützen. Als Agrarchemikalien kommen beispielsweise Defoliationsmittel, Desiccationsmittel, Mittel zum Schutz gegen Frostschäden und Pflanzenschutzmittel, wie beispielsweise Insektizide, Fungizide, Bakterizide, Nematozide und insbesondere Herbizide in Betracht. Die Agrarchemikalien können verschiedenen Stoffklassen angehören. Herbizide können beispielsweise zu einer der folgenden Stoffklassen gehören: Triazine und Triazinone; Harnstoffe wie beispielsweise 1 - (Benzthiazol - 2 - yl) - 1,3 - dimethylharnstoff ("Methabenzthiazuron") oder insbesondere Phenylharnstoffe, vor allem 3 - (4 - Isopropylphenyl) - 1,1 - dimethylharnstoff ("Isoproturon") oder Sulfonylharnstoffe: Carbamate und Thiocarbamate; Halogenacetanilide, insbesondere Chloracetanilide; Chloracetamide; Halogenphenoxyessigsäureester; Diphenyläther, wie beispielsweise substituierte Phenoxyphenoxyessigsäureester und -amide und substituierte Phenoxyphenoxypropionsäureester und -amide; substituierte Pyridyloxyphenoxyessigsäureester und -amide und substituierte Pyridyloxyphenoxypropionsäureester und -amide, insbesondere 2 - [4 - (3,5 - Dichlorpyridyl - 2 - oxy) - phenoxy] - propionsäure - 2 - propinylester und 2 - [4 - (5 - Trifluormethylpyridyl - 2 - oxy) - phenoxy] - propionsäure - n - butylester; Benzoesäurederivate; Nitroaniline; Oxadiazolone; Phosphate; und Pyrazole.

Im einzelnen kommen beispielsweise folgende Substanzen in Betracht: Triazine und Triazinone: 2,4 - Bis(isopropylamino) - 6 - methylthio - 1,3,5 - triazin ("Prometryn"), 2,4 - bis(äthylamino) - 6 - methylthio - 1,3,5 - triazin ("Simetryn"), 2 - (1',2' - Dimethylpropylamino) - 4 - äthylamino - 6 - methylthio - 1,3,5 - triazin ("Dimethametryn") 4 - Amino - 6 - tert.butyl - 4,5 - di - hydro - 3 - methylthio - 1,2,4 - triazin - 5 - on ("Metribuzin"), 2 - Chlor - 4 - äthylamino - 6 - isopropylamino - 1,3,5 - triazin ("Atrazin"), 2 - Chlor - 4,6 - bis - (äthylamino) - 1,3,5 - triazin ("Simazin"), 2 - tert.Butylamino - 4 - chlor - 6 - äthylamino - 1,3,5 - triazin ("Terbuthylazin"), 2 - tert.Butylamino - 4 - äthylamino - 6 - methoxy - 1,3,5 - triazin ("Terbumeton"), 2 - tert.Butylamino - 4 - äthylamino - 6 - methylthio - 1,3,5 - triazin ("Terbutryn"), 2 - Aethylamino - 4 - isopropylamino - 6 - methylthio - 1,3,5 - triazin ("Ametryn");

Harnstoffe: 1 - (Benzothiazol - 2 - yl) - 1,3 - dimethylharnstoff; Phenylharnstoffe wie beispielsweise 3 - (3 - Chlor - p - tolyl) - 1,1 - dimethylharnstoff ("Clortoluron"), 1,1 - Dimethyl - 3 - (ααα - trifluor - m - tolyl) - harnstoff ("Fluometuron"), 3 - (4 - Brom - 3 - chlorphenyl) - 1 - methoxy - 1 - methylharnstoff ("Chlorbromuron"), 3 - (4 - Bromphenyl) - 1 - methoxy - 1 - methylharnstoff ("Metobromuron"), 3 - (3,4 - Dichlorphenyl) - 1 - methoxy - 1 - methylharnstoff ("Linuron"), 3 - (4 - Chlorphenyl) - 1 -

methoxy - 1 - methylharnstoff ("Monolinuron"), 3 - (3,4 - Dichlorphenyl) - 1,1 - dimethylharnstoff ("Diuron"), 3 - (4 - Chlorphenyl) - 1,1 - dimethylharnstoff ("Monuron"), 3 - (3 - Chlor - 4 - methoxyphenyl) - 1,1 - dimethylharnstoff ("Metoxuron"); Sulfonylharnstoffe wie beispielsweise N - (2-Chlorphenylsulfonyl) - N' - (4 - methoxy - 6 - methyl - 1,3,5 - triazin - 2 - yl) - harnstoff, N - (2 - Methoxycarbonylphenylsulfonyl) - N' - (4,6 - dimethylpyrimidin - 2 - yl) - harnstoff, N - (2,5 - Dichlorphenylsulfonyl) - N' - (4,6 - dimethoxypyrimidin - 2 - yl) - harnstoff, N - [2 - (2 - butenyloxy) - phenylsulfonyl] - N' - (4 - methoxy - 6 - methyl - 1,3,5 - triazin - 2 - yl) - harnstoff sowie die in den EP—A—44808 und EP—A—44809 genannten Sulfonylharnstoffe;

Carbamate und Thiocarbamate: N - (3',4' - Dichlorphenyl) - propionanilid ("Propanil"), S-Benzyl - diäthyl - thiocarbamat, S - 4 - Chlorbenzyl - diäthylthiocarbamat ("Benthiocarb"), S - Aethyl - N,N - hexamethylen - thiocarbamat ("Molinate"), S - Aethyl - dipropyl - thiocarbamat ("EPTC"), N,N - di - sec. - Butyl - S - benzyl - thiocarbamat, S - (2,3 - Dichlorallyl) - di - isopropyl - thiocarbamat ("Diallate"), 1 - (Propylthiocarbonyl) - decahydro - chinaldin, S - Aethyl - di - isobutyl - thiocarbamat ("Butylate");

Chloracetanilide: 2 - Chlor - 2',6' - diäthyl - N - (2" - n - propoxyäthyl) - acetanilid ("Propalochlor"), 2 - Chlor - 6' - äthyl - N - (2" - methoxy - 1" - methyläthyl) - acet - o - toluidid ("Metolachlor"), 2 - Chlor - 2',6' - diäthyl - N - (butoxymethyl)acetanilid ("Butachlor"), 2 - Chlor - 6' - äthyl - N - (àthoxymethyl)acet - o - toluidid ("Acetochlor"), 2 - Chlor - 6' - äthyl - N - (2" - propoxy - 1" - methyläthyl)-acet - o - toluidid, 2 - Chlor - 2',6' - dimethyl - N - (2" - methoxy - 1" - methyläthyl)acetanilid, 2 - Chlor-2',6' - dimethyl - N - (2" - methoxy - äthyl)acetanilid ("Dimethachlor"), 2 - Chlor - 2',6' - diäthyl - N - (pyrazol - 1 - ylmethyl)acetanilid, 2 - Chlor - 6' - äthyl - N - (pyrazol - 1 - ylmethyl)acet - o - toluidid, 2 - Chlor - 6' - äthyl - N - (3,5 - dimethyl - pyrazol - 1 - ylmethyl)acet - o - toluidid, 2 - Chlor - 6' - athyl - N - (2" - butoxy - 1" - methyläthyl)acet - o - toluidid ("Metazolachlor"), 2 - Chlor - 6' - äthyl - N - (2" - butoxyl - 1" - (methyläthyl)acet - o - toluidid und 2 - Chlor - 2' - trimethylsilyl - N - (butoxymethyl) - acetanilid;

Chloracetamide: N - [1 - Isopropyl - 2 - methylpropen - 1 - yl - (1)] - N - (2' - methoxyäthyl) - chloracetamid;

Diphenyläther und Nitrodiphenyläther: 2,4 - Dichlorphenyl - 4' - nitrophenyläther ("Nitrofen"), 2 - Chlor - 1 - (3' - äthoxy - 4' - nitrophenoxy) - 4 - trifluormethyl-benzol ("Oxyfluorfen"), 2',4' - Dichlorphenyl - 3 - methoxy - 4 - nitrophenyl - äther ("Chlormethoxynil"), 2 - [4' - (2",4" - Dichlorphenoxy) - phenoxy)propionsäure - methylester, N - (2' - Phenoxyäthyl) - 2 - [5'(2" - chlor - 4" - trifluormethylphenoxy) - phenoxy] - propionsäureamid, 2 - [2 - Nitro - 5 - (2 - chlor - 4 - trifluormethylphenoxy) - phenoxy] - propionsäure - 2 - methoxyäthylester; 2 - Chlor - 4 - trifluormethylphenyl - 3' - oxazolin - 2' - yl - 4' - nitrophenyläther;

Benzoesäurederivative: Methyl - 5 - (2',4' - dichlorphenoxy) - 2 - nitrobenzoat ("Bifenox"), 5 - (2' - Chlor - 4' - trifluormethylphenoxy) - 2 - nitrobenzoesäure ("Acifluorfen"), 2,6 - Dichlorbenzonitril ("Dichlobenil");

Nitroaniline: 2,6 - Dinitro - N,N - dipropyl - 4 - trifluormethylanilin ("Trifluralin"), N - (1' - Aethylpropyl) - 2,6 - dinitro - 3,4 - xylidin ("Pendimethalin");

Oxadiazolone: 5 - tert. - Butyl - 3 - (2',4' - dichlor - 5' - isopropoxyphenyl) - 1,3,4 - oxadiazol - 2 - on ("Oxadiazon");

Phosphate: S - 2 - Methylpiperidino - carbonylmethyl - O,O - dipropyl - phosphorodithioat ("Piperophos");

Pyrazole: 1,3 - Dimethyl - 4 - (2',4' - dichlorbenzoyl) - 5 - (4' - tolylsulfonyloxy) - pyrazol;

2 - [1 - (Äthoxyimino) - butyl] - 5 - [2 - (äthylthio) - propyl] - 3 - hydroxy - 2 - cyclohexen - 1 - on und das Natriumsalz von 2 - [1-(N - Allyloxyamino) - butyliden] - 5,5 - dimethyl - 4 - methoxycarbonyl - cyclohexan - 1,3 - dion.

Ganz besonders hervorzuheben ist jedoch die ausgezeichnete Schutzwirkung gegen schädigende Wirkungen von Halogenacetaniliden, insbesondere Chloracetaniliden, vor allem 2 - Chlor - 2',6' - diäthyl - N - (2"-propoxyäthyl) - acetanilid und 2 - Chlor - 6' - äthyl - N - (2" - methoxy - 1" - methyläthyl) - acet - o - toluidid. Ebenfalls herausragend ist die Wirkung gegen die Thiocarbamate S - Benzyl - diäthyl - thiocarbamat und S - 4 - Chlorbenzyl - diäthyl - thio - carbamat sowie gegen das Pyridyloxyphenoxyessigsäureester-Derivat 2 - [4 - (3,5 - Dichlorpyridyl - 2 - oxy) - phenoxy] - propionsäure - 2 - propinylester.

Als Kulturpflanzen, welche durch Cyclopropancarbonsäurederivate der Formel I gegen Agrarchemikalien geschützt werden können, kommen insbesondere diejenigen in Betracht, die auf dem Nahrungs- oder Textilsektor von Bedeutung sind, wie beispielsweise Kulturhirse, Reis, Mais, Getriedearten (Weizen, Roggen, Gerste, Hafer), Baumwolle, Zuckerrüben, Zuckerrohr und Soja.

Die Verbindungen der Formel I sind insbesondere hervorragend dazu geeignet, Reispflanzen gegen schädigende Wirkungen von Chloracetaniliden zu schützen.

Ein geeignetes Verfahren zum Schützen von Kulturpflanzen unter Verwendung von Verbindungen der Formel I besteht darin, dass man Kulturpflanzen, Teile dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz der Agrarchemikalie erfolgen. Als

Pflanzenteile kommen insbesondere diejenigen in Betracht, die zur Neubildung einer Pflanze befähigt sind, wie beispielsweise Samen, Früchte, Stengelteile und Zweite (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit Kulturpflanzen bewachsenen oder die ausgesäten Bodenareale, wie auch die zur Bebauung mit Kulturpflanzen bestimmten Böden.

Die zu applizierende Aufwandmenge Antidot im Verhältnis zur Agrarchemikalie richtet sind weitgehend nach der Anwendungsart. Bei einer Feldbehandlung, welche entweder unter Verwendung einer Tankmischung oder durch getrennte Applikation von Agrarchemikalie und Antidot durchgeführt wird, liegt in der Regel ein Verhältnis von Antidot zu Agrarchemikalie von 1:100 bis 10:1, bevorzugt 1:5 bis 8:1, und insbesondere 1:1, vor.

Dagegen werden bei der Samenbeizung und ähnlichen Einsatzmethoden weit geringere Mengen Antidot im Verhältnis zur Aufwandmenge an Agrarchemikalie/ha Anbaufläche benötigt. Bei der Samenbeizung werden in der Regel 0,1 bis 10 g Antidot/kg Samen, bevorzugt 1 bis 2 g, appliziert. Wird das Gegenmittel kurz vor der Aussaat unter Samenquellung appliziert, so werden zweckmässigerweise Antidot-Lösungen verwendet, welche den Wirkstoff in einer Konzentration von 1 bis 10 000 ppm, bevorzugt 100 bis 1000 ppm, enthalten.

Die Verbindungen der Formel I können für sich allein oder zusammen mit inerten Zusatzstoffen und/oder den zu antagonisierenden Agrarchemikalien zur Anwendung gelangen.

Die vorliegende Anmeldung betrifft daher auch Mittel, welche Verbindungen der Formel I und inerte Zusatzstoffe und/oder zu antagonisierende Agrarchemikalien, insbesondere Pflanzenschutzmittel und vor allem Herbizide, enthalten.

Zur Applikation werden die Verbindungen der Formel I oder Kombinationen von Verbindungen der Formel I mit zu antagonisierenden Agrarchemikalien zweckmässigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d.h. die den Wirkstoff der Formel I oder eine Kombination von Wirkstoff der Formel I mit zu antagonisierender Agrarchemikalie und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch der zu antagonisierenden Agrarchemikalie nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von

natürlichen Fettsäuregemischen, die z.B. aus Kokosnuss- oder Talgöl gewonnen werden können, genannt. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Aethylenoxid-Addukktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminpolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood New Jersey, 1980 Sisely and Wood, "Encyclopedia of Surface Active Agents", Chemical Publishing Co., Inc. New York, 1980

Die agrochemischen Zubereitungen enthalten in der Regel 0,1 bis 99%, insbesondere 0,1 bis 95%, Wirkstoff der Formel I, 99,9 bis 1%, insbesonderes 99,8 bis 5%, eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltende Mittel zum Schützen von Kulturpflanzen gegen schädigende Wirkungen von aggressiven Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionkonzentrat des Wirkstoffs der Formel I nach der Methode a) (Nassbeizung).

c) Beizung durch Tauchen des Saatgutes in eine Brühe mit 50—3200 ppm Wirkstoff der Formel I während 1 bis 72 Stunden und gegebenenfalls nachfolgendes Trocknen der Samen (Tauchbeizung).

Die Beizung des Saatgutes oder die Bahandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:10) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Herstellung von Verbindungen der Formel I erfolgt, indem man

a) eine Verbindung der Formel II

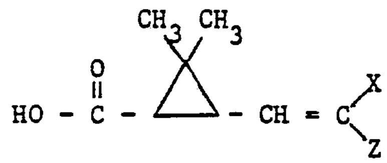

(II),

worin X und Z die für Formel I angegebenen Bedeutungen haben, mit einer Verbindung der Formel III

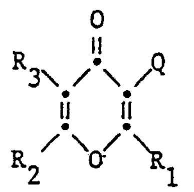

(III),

worin $R_1$, $R_2$ und $R_3$ die für Formel I angegebenen Bedeutungen haben und Q für einen nucleofugen Rest steht, umsetzt oder

b) eine Verbindung der Formel IV

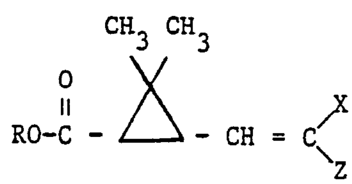

(IV),

worin X und Z die für Formel I angegebenen Bedeutungen haben und R für einen organischen Rest steht, mit einer Verbindung der Formel V

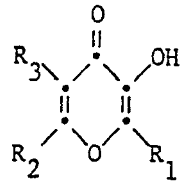

(V),

worin $R_1$, $R_2$ und $R_3$ die für Formel I angegebenen Bedeutungen haben, umsetzt oder

c) eine Verbindung der Formel VI

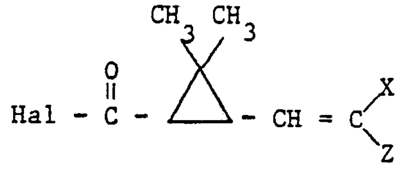

(VI),

worin X und Z die für Formel I angegebenen Bedeutungen haben und Hal für ein Halogenatom steht, mit einer Verbindung der Formel V umsetzt.

In der Verfahrensvariante a) kann Q als nucleofuger Rest beispielsweise für ein Halogenatom oder die Sulfonatgruppe $—OSO_2R'$ stehen, worin R' beispielsweise für einen gegebenenfalls durch $C_1—C_4$-Alkyl mono- oder disubstituierten Phenyl- oder Naphthylrest oder einen aliphatischen Rest mit maximal 6 Kohlenstoffatomen, bevorzugt $C_1—C_3$-Alkyl, steht.

In der Verfahrensvariante b) kommt als organischer Rest R vor allem $C_1—C_6$-Alkyl, vorzugsweise $C_1—C_3$-Alkyl, in Betracht.

In den Ausgangsprodukten der vorgenannten Umsetzungen steht Halogen für Fluor, Chlor, Brom oder Jod.

Die Umsetzungen gemäss den Verfahrensvarianten a) und c) werden in Gegenwart einer Base durchgeführt. Als geeignete Basen sind beispielsweise schwach basische anorganische Salze, wie beispielsweise Kaliumcarbonat, oder bevorzugt organische Basen wie beispielsweise Pyridin, Chinolin, Methylpiperidin, Dimethylanilin oder Triäthylamin, anzusehen.

Die Umesterung gemäss Verfahrensvariante b) wird zweckmässigerweise in Gegenwart eines basischen Umesterungskatalysators durchgeführt. Als Katalysatoren eignen sich beispielsweise Natrium- oder Kaliumhydroxid oder Natrium- oder Kaliumalkoholate, insbesondere -alkylate mit 1 bis 4 Kohlenstoffatomen, wie beispielsweise Natriumäthylat.

Die Umsetzungen gemäss Verfahrensvarianten a), b) und c) können in einem Temperaturbereich von −20 bis +200°C durchgeführt werden. Bei der Verfahrensvariante b) werden die Reaktionsteilnehmer zweckmässigerweise in Gegenwart eines basischen Umesterungskatalysators erhitzt. Die Umsetzung gemäss Verfahrensvariante c) lässt sich leicht bei Raumtemperatur durchführen. Zweckmässigerweise wählt man für diese Umsetzung einen Bereich zwischen −20° und +25°C.

Die in den vorgenannten Verfahrensvarianten a), b) und c) verwendeten Ausgangsprodukte sind bekannt oder lassen sich analog bekannten Verfahren herstellen.

Die nachfolgenden Beispiele dienen zur Illustration der Erfindung.

Beispiel 1:

2-(2,2-Dichlorvinyl)-3,3-dimethyl-cyclopropancarbonsäure-(2-methyl-4-pyron-3-yl)-ester

3,78 g 3 - Hydroxy - 2 - methyl - 4 - pyron und 7,5 g 2 - (2,2 - Dichlorvinyl) - 3,3 - dimethyl - cyclopropancarbonsäurechlorid (85% cis, 15% trans) werden in 100 ml Toluol gelöst und 2,9 ml Pyridin in 10 ml Toluol bei 15°C über einen Zeitraum von 10 Minuten zugetropft. Nach Zugabe von 0,1 g 4-Dimethylaminopyridin wird die Reaktionsmischung 6 Stunden bei 50°C gerührt. Die Lösung wird gekühlt und nacheinander mit Wasser, 2n HCl und $NaHCO_3$ (5%ig) gewaschen, getrocknet und eingedampft. Man erhält 8,3 g 2 - (2,2 - Dichlorvinyl) - 3,3 - dimethyl - cyclopropancarbonsäure - (2 - methyl - 4 - pyron - 3 - yl) - ester als cis-trans-Isomerengemisch mit 85% cis-Form und 15% trans-Form. Smp. <50°C.

Durch Umkristallisation aus Aether/Pentan erhält man die reine cis-Form von 2 - (2,2 - Dichlorvinyl) - 3,3 - dimethyl - cyclopropancarbonsäure - (2 - methyl - 4 - pyron - 3 - yl) - ester. Smp. 79—83°C.

Analog einer der vorstehend beschriebenen Methoden lassen sich auch die folgenden, in der Tabelle 1 zusammen mit der Verbindung aus dem vorstehenden Beispiel aufgeführten Verbindugen der Formel I herstellen:

TABELLE 1

| Nr. | $R_1$ | $R_2$ | $R_3$ | X | Z | Konfiguration % cis | Konfiguration % trans | physikalische Daten |
|---|---|---|---|---|---|---|---|---|
| 1 | $CH_3$ | H | H | Cl | Cl | 85 | 15 | Smp. <50°C |
| 2 | $CH_3$ | H | H | Cl | Cl | 100 | 0 | Smp. 79—83°C |
| 3 | $CH_3$ | H | H | Cl | Cl | 0 | 100 | Smp. 136—137°C |
| 4 | $C_2H_5$ | H | H | Cl | Cl | 85 | 15 | $n_D^{21}$ 1,5385 |
| 5 | $CH_3$ | H | H | Br | Br | Gemisch | | |
| 6 | $CH_3$ | H | H | F | F | Gemisch | | Smp. 85—86°C |
| 7 | $CH_3$ | H | H | Cl | Cl | 100 | 0 | |
| 8 | H | $—CH_2OH$ | H | Cl | Cl | 85 | 15 | |
| 9 | H | $—CH_2SCN$ | H | Cl | Cl | 85 | 15 | |
| 10 | H | $—CH_2OCOCH_3$ | H | Cl | Cl | 85 | 15 | |

Formulierungsbeispiele für flüssige Wirkstoffe der Formel I
(%=Gewichtsprozent)

| 2. Emulsions-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykol-äther (36 Mol AeO) | 5% | — | — |
| Tributylphenol-polyäthylen-glykoläther (30 Mol AeO) | — | 12% | 4% |
| Cyclohexanon | — | 15% | 20% |
| Xylolgemisch | 65% | 25% | 20% |

Aus solchen Konzentraten koinnen durch Verdünnen mit Wasser Emulsionen jeder gewünschten Konzentration hergestellt werden.

| 3. Lösungen | a) | b) | c) | d) |
|---|---|---|---|---|
| Wirkstoff aus Tabelle 1 | 80% | 10% | 5% | 95% |
| Aethylenglykol-monomethyläther | 20% | — | — | — |
| Polyäthylenglykol MG 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

Die Lösungen sind zur Anwendung in Form kleinster Tröpfchen geeignet.

| 4. Granulate | a) | b) |
|---|---|---|
| Wirkstoff aus Tabelle 1 | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| 5. Stäubemittel | a) | b) |
|---|---|---|
| Wirkstoff aus Tabelle 1 | 2% | 5% |
| Hochdisperse Kieselsäure | 1% | 5% |
| Talkum | 97% | — |
| Kaolin | — | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 6. Spritzpulver | a) | b) | c) |
|---|---|---|---|
| Wirkstoff aus Tabelle 1 | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykoläther (7—8 Mol AeO) | — | 2% | — |
| Hochdisperse Kieselsäure | 5% | 10% | 10% |
| Kaolin | 62% | 27% | — |

Der Wirkstoff mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 7. Emulsions-Konzentrat | |
|---|---|
| Wirkstoff aus Tabelle 1 | 10% |
| Octylphenolpolyäthylenglykoläther (4—5 Mol AeO) | 3% |
| Ca-Dodecylbenzolsulfonat | 3% |
| Ricinusölpolyglykoläther (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 8. Stäubemittel | a) | b) |
|---|---|---|
| Wirkstoff aus Tabelle 1 | 5% | 8% |
| Talkum | 95% | — |
| Kaolin | — | 92% |

Man erhält anwendungsfertige Stäubemittel, indem der Wirkstoff mit den Trägerstoffen vermischt und auf einer geeigneten Mühle vermahlen wird.

| 9. Extruder-Granulat | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschlessend im Luftstrom getrocknet.

| 10. Umhüllungs-Granulat | |
|---|---|
| Wirkstoff aus Tabelle 1 | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 11. Suspensions-Konzentrat | |
|---|---|
| Wirkstoff aus Tabelle 1 | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele
Beispiel 12:
Versuch mit Antidot und Herbizid an verpflanztem Reis; Applikationsmethode: Tankmischung

Reispflanzen werden bis zum 1 1/2-2-Blattstadium in Erde angezogen. Die Pflanzen werden dann

büschelweise (jeweils 3 Pflanzen zusammen) in Container (47 cm lang, 29 cm breit und 24 cm hoch) in sandigen Lehm verpflanzt. Die Bodenoberfläche wird anschliessend 1,5—2 cm hoch mit Wasser beschichtet. 2—3 Tage nach dem Verpflanzen wird als Herbizid 2 - Chlor - 2',6' - diäthyl - N - (2" - propoxyäthyl) - acetanilid ("Pretilachlor") zusammen mit 2 - (2,2 - Dichlorvinyl) - 3,3 - dimethyl - cyclopropancarbonsäure - (2 - methyl - 4 - pyron - 3 - yl) - ester als Antidot als Tankmischung direkt ins Wasser appliziert. 24 Tage nach dem Verpflanzen wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (=100% Wachstum). Die Ergebnisse sind in Tabelle 2 dargestellt:

TABELLE 2:

| Aufwandmenge kg/ha | | relative Schutzwirkung in % |
|---|---|---|
| Herbizid | Antidot | |
| 1,0 | 1,0 | 50 |
| 0,75 | 0,75 | 50 |
| 0,5 | 0,5 | 38 |

Beispiel 13:

Versuch mit Antidot und Herbizid an verpflanztem Reis; Applikationsmethode: Wurzelbehandlung

Reispflanzen der Sorte Yamabiko werden bis zum 1 1/2-2-Blattstadium in Erde angezogen und dann ausgewaschen. Die Pflanzen werden büschelweise (jeweils 3 Pflanzen zusammen) während 15 bis 60 Minuten nur mit den Wurzeln in eine Lösung von 2 - (2,2 - Dichlorvinyl) - 3,3 - dimethylcyclopropancarbonsäure - (2 - methyl - 4 - pyron - 3 - yl) - ester als Antidot mit einer Konzentration von 10 oder 100 ppm getaucht. Anschliessend werden diese Pflanzen in Container (47 cm lang, 29 cm breit und 24 cm hoch) in sandigen Lehm verpflanzt. Die Bodenoberfläche wird 1,5—2 cm hoch mit Wasser beschichtet. 2—3 Tage nach dem Verpflanzen wird 2 - Chlor - 2',6' - diäthyl - N - (2" - propoxyäthyl) - acetanilid ("Pretilachlor") als Herbizid direkt ins Wasser appliziert. 10 und 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (=100% Wachstum). Die Ergebnisse sind in Tabelle 3 dargestellt.

TABELLE 3

| Aufwandmenge | | relative Schutzwirkung in % |
|---|---|---|
| Herbizid, kg/ha | Antidot, ppm | |
| 0,75 | 100 | 38 |
| | 10 | 25 |
| 0,5 | 100 | 25 |
| | 10 | 12,5 |

Beispiel 14:

Versuch mit Antidot und Herbizid as in Wasser gesätem Reis; Applikationsmethode: Tankmischung

Reissamen werden während 48 Stunden in Wasser vorgequollen. Plastik-Container (25 cm lang, 17 cm breit und 12 cm hoch) werden mit Erde gefüllt, in welche die vorgequollenen Reissamen eingesät werden. Anschliessend wird als Herbizid 2 - Chlor - 2',6' - diäthyl - N - (2" - propoxyäthyl) - acetanilid ("Pretilachlor") zusammen mit 2 - (2,2 - Dichlorvinyl) - 3,3 - dimethyl - cyclopropancarbonsäure - (2 - methyl - 4 - pyron - 3 - yl) - ester als Antidot als Tankmischung versprüht. Der Wasserstand wird entsprechend dem Wachstum der Reispflanzen sukzessive erhöht. 18 Tage nach der Aussaat wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (=100% Wachstum). Die Ergebnisse sind in Tabell 4 dargestellt:

TABELLE 4

| Aufwandmenge kg/ha | | relative Schutzwirkung in % |
|---|---|---|
| Herbizid | Antidot | |
| 1,0 | 1,0<br>0,5<br>0,25 | 38<br>25<br>25 |
| 0,75 | 0,75<br>0,375<br>0,187 | 50<br>38<br>12,5 |
| 0,5 | 0,5<br>0,25<br>0,125<br>0,06 | 50<br>50<br>63<br>25 |

Beispiel 15:

Versuch mit Antidot und Herbizid an trocken gesätem Reis; Applikationsmethode: Tankmischung

Reissamen der Sorte IR-36 werden in Container (47 cm lang, 29 cm breit und 24 cm hoch) eingesät, bedeckt und leicht festgesdrückt. Dann wird als Herbizid 2 - Chlor - 2',6' - diäthyl - N - (methoxymethyl) - acetanilid ("Alachlor") zusammen mit 2 - (2,2 - Dichlorvinyl) - 3,3 - dimethylcyclopropancarbonsäure - (2 - methyl - 4 - pyron - 3 - yl) - ester als Antidot als Tankmischung versprüht. Etwa 20 Tage nach der Aussaat (3-Blattstadium der Reispflanzen) wird die Bodenoberfläche 4 cm hoch mit Wasser beschichtet. 30 Tage nach der Aussaat wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (=100% Wachstum). Die Ergebnisse sind in Tabelle 5 dargestellt.

TABELLE 5

| Aufwandmenge kg/ha | | relative Schutzwirkung in % |
|---|---|---|
| Herbizid | Antidot | |
| 0,25 | 0,25 | 38 |
| 0,125 | 0,125 | 25 |

Beispiel 16:

Versuch mit Antidot und Herbizid an trocken gesätem Reis; Applikationsmethode: Saatbeizung

Reissamen werden mit 2 - (2,2 - Dichlorvinyl) - 3,3 - dimethyl - cyclopropancarbonsäure - (2 - methyl - 4 - pyron - 3 - yl) - ester als Antidot in einem Glasbehälter gemischt. Samen und Antidot werden durch Schütteln und Rotation gut zusammengemischt. Dann werden Container (47 cm lang, 29 cm breit und 24 cm hoch) mit sandiger Lehmerde gefüllt und die gebeizten Samen werden eingesät. Nach dem Bedecken der Samen wird als Herbizid 2 - Chlor - 6' - äthyl - N - (2" - methoxy - 1" - methyläthyl) - acet - o - toluidid ("Metolachlor") in einer verdünnten Lösung auf die Bodenoberfläche versprüht. Etwa 20 Tage nach der Aussaat (3-Blattstadium der Reispflanzen) wird die Bodenoberfläche 4 cm hoch mit Wasser beschichtet. 30 Tage nach der Herbizidapplikation wird die Schutzwirkung des Antidots in Prozent bonitiert. Als Referenzen dienen dabei die mit dem Herbizid allein behandelten Pflanzen (keine Schutzwirkung) sowie die vollständig unbehandelte Kontrolle (=100% Wachstum). Die Ergebnisse sind in Tabelle 6 dargestellt:

TABELLE 6

| Aufwandmenge | | relative Schutzwirkung in % |
|---|---|---|
| Herbizid, kg/ha | Andidot, g/kg Samen | |
| 1,0 | 4 | 38 |
| 1,0 | 1 | 25 |
| 0,75 | 4 | 38 |
| 0,5 | 1 | 25 |

Beispiel 17:
Samenquellung Reis, Herbizid im Vorauflaufverfahren.

Reissamen werden 48 Stunden mit Lösungen der als Antidot zu prüfenden Substanz in einer Konzentration von 100 ppm getränkt. Man lässt die Samen dann etwa zwei Stunden trocknen, bis sie nicht mehr kleben. Plastikbehälter (Länge×Breite×Höhe=25×17×12 cm) werden bis 2 cm unter dem Rand mit sandigem Lehm gefüllt. Die vorgequollenen Samen werden auf die Bodenoberfläche des Behälters gesät und nur ganz schwach mit Erde bedeckt. Die Erde wird in einem feuchten (nicht sumpfigen) Zustand gehalten. Nun wird das Herbizid 2 - Chlor - 2',6' - diäthyl - N - [2" - (n - propoxy) - äthyl] - acetanilid in verdünnter Lösung auf die Bodenoberfläche versprüht. Der Wasserstand wird entsprechend dem Wachstum der Pflanzen sukzessive erhöht. 18 Tage nach der Herbizidapplikation wird die Schutzwirkung des Antidots in Prozent bonitiert. Als Referenz dienen dabei die mit dem Herbizid allein behandelten Pflanzen (keine Schutzwirkung) sowie die vollständig unbehandelte Kontrolle (=100% Wachstum). Die Ergebnisse zeigt die folgende Tabelle:

TABELLE 7

| Antidot Verbindung Nr. | Antidot ppm | Herbizid kg AS/ha | relative Schutzwirkung in % |
|---|---|---|---|
| 1 | 100 | 0,25 | 63 |
| 3 | 100 | 0,25 | 25 |
| 4 | 100 | 0,25 | 25 |

Beispiel 18:
Sattbeizung Reis, Herbizid im Vorauflaufverfahren.

Reissamen der Sorte IR-36 werden mit 2 - (2,2 - Dichlorvinyl) - 3,3 - dimethylcyclopropancarbonsäure - (2 - methyl - 4 - pyron - 3 - yl) - ester als Antidot in einen Glasbehälter gegeben und durch Schütteln und Rotation gut durchgemischt. Plastikbehälter (Länge×Breite×Höhe=47×29×24 cm) werden mit sandiger Lehmerde gefüllt und die gebeizten Samen eingesät. Nach dem Bedecken des Samens mit Erde wird das Herbizid 2 - Chlor - 6' - äthyl - N - (2" - methoxy - 1" - methyläthyl) - acet - o - toluidid ("Metolachlor") auf die Bodenoberfläche versprührt. 18 Tage nach der Aussaat wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (=100% Wachstum). Die Ergebnisse zeigt die folgende Tabelle:

TABELLE 8

| Antidot g AS/kg Samen | Herbizid kg AS/ha | relative Schutzwirkung in % |
|---|---|---|
| 4 | 1,0 | 38 |
| 4 | 0,75 | 38 |

Beispiel 19:

Versuch mit Antidot und Herbizid an verfplanztem Reis Applikationsmethode: Einmischen in den Boden (ppi-Methode).

Das Antidot 2 - (2,2 - Dichlorvinyl) - 3,3 - dimethyl - cyclopropancarbonsäure - (2 - methyl - 4 - pyron - 3 - yl) - ester wird in die Erde in Anzuchtschalen in einer Konzentration von 10 und 100 ppm eingemischt. Nach Ablauf von 2 Tagen werden Reispflanzen bis zum 1½—2-Blattstadium in den behandelten Anzuchtschalen angezogen. Die Pflanzen werden dann büschelweise (jeweils 3 Pflanzen zusammen) in Container (Länge×Breite×Höhe=47×29×24 cm) in sandigen Lehm verpflanzt. Die Bodenoberfläche wird 1,5—2 cm hoch mit Wasser beschichtet. 2—3 Tage nach dem Verpflanzen wird das Herbizid S - 4 - Chlorbenzyl - diäthyl - thiocarbamat ("Benthiocarb") direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (=100% Wachstum). Die Ergebnisse sind in der folgenden Tabelle dargestellt:

TABELLE 9

| Antidot, ppm | Herbizid, kg/ha | relative Schutzwirkung in % |
|---|---|---|
| 100 | 8 | 50 |
| 10 | 8 | 25 |

Beispiel 20:

Versuch mit Antidot und Herbizid an verpflanztem Reis Applikationsmethode: Tauchbad (Drench-Methode).

Reispflanzen der Sorte Yamabiko werden bis zum 1½—2-Blattstadium in Anzuchtschalen angezogen. 1—2 Tage vor dem Verpflanzen wird die ganze Anzuchtscale mit den Reispflanzen in eine grössere Schale, welche eine wässrige Lösung von 2 - (2,2 - Dichlorvinyl) - 3,3 - dimethyl - cyclopropancarbonsäure - (2 - methyl - 4 - pyron - 3 - yl) - ester als Antidot enthält, getaucht.

Die Pflanzen werden dann büschelweise (jeweils 3 Pflanzen) in Container (Länge×Breite×Höhe=47×29×24 cm) in sandigen Lehm verpflanzt. Die Bodenoberfläche wird 1,5—2 cm hoch mit Wasser beschichtet. 2—3 Tage nach dem Verpflanzen wird das Herbizid S - 4 - Chlorbenzyl - diäthyl - thiocarbamat direkt ins Wasser appliziert. 24 Tage nach dem Verpflanzen wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Bei diesem Test wird das folgende Ergebnis erhalten:

TABELLE 10

| Antidot, ppm | Herbizid, kg/ha | relative Schutzwirkung in % |
|---|---|---|
| 100 | 8 | 25 |

Beispiel 21:

Tankmischung im Nachauflaufverfahren in Weizen.

Weizensamen der Sorte "Farnese" werden in Plastiktöpfe (oberer Durchmesser 11 cm), die 0,5 1 Erde enthalten, im Gewächshaus ausgesät. Nach dem Bedecken der Samen mit Erde wird die als Antidot zu prüfende Substanz zusammen mit dem Herbizid 2 - [4 - (3,5 - Dichlorpyridyl - 2 - oxy) - phenoxy] - propionsäure - 2 - propinylester als Tankmischung im Nachauflaufverfahren appliziert. 20 Tage nach der Applikation wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle:

TABELLE 11

| Antidot Verbindung Nr. | Antidot kg AS/ha | Herbizid kg AS/ha | relative Schutzwirkung in % |
|---|---|---|---|
| 1 | 1,5 | 0,75 | 50 |
| 4 | 1,5 | 0,75 | 50 |

Beispiel 22:
Tankmischung im Vorauflaufverfahren in Mais.

Maissamen der Sorte "LG5" werden in Plastiktöpfe (oberer Durchmesser 11 cm), die 0,5 1 Erde enthalten, im Gewächshaus ausgesät. Nach dem Bedecken der Samen mit Erden wird als Antidot 2 - (2,2 - Dichlorvinyl) - 3,3 - dimethyl - cyclopropancarbonsäure - (2 - methyl - 4 - pyron - 3 - yl) - ester zusammen mit dem Herbizid 2 - Chlor - 2',6' - dimethyl - N - (2" - methoxy - 1" - methyläthyl) - acetanilid als Tankmischung auf die Bodenoberfläche appliziert. 21 Tage nach der Applikation wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle:

TABELLE 12

| Antidot kg AS/ha | Herbizid kg AS/ha | relative Schutzwirkung in % |
|---|---|---|
| 2,0 | 2,0 | 25 |
| 1,0 | 2,0 | 38 |

Beispiel 23:
Versuch mit Herbizid und Antidot an Sorghum (Hirse) Applikationsmethode: Tankmischung im Vorauflauf.

Plastikbehälter (Länge×Breite×Höhe=47×17×12 cm) werden mit sandiger Lehmerde gefühllt und Sorghumsamen der Sorte "Funk G 522" werden eingesät. Nach dem Bedecken der Samen wird 2 - (2,2 - Difluorvinyl) - 3,3 - dimethyl - cyclopropancarbonsäure - (2 - methyl - 4 - pyron - 3 - yl) - ester als Antidot zusammen mit dem Herbizid 2 - Chlor - 6' - äthyl - N - (2" - methoxy - 1" - methyläthyl) - aceto - o - toluidid ("Metolachlor") in verdünnter Lösung als Tankmischung auf die Bodenoberfläche gesprüht. 30 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100% Wachstum). Das Ergebnis ist wie folgt:

TABELLE 13

| Antidot kg/ha | Herbizid kg/ha | relative Schutzwirkung in % |
|---|---|---|
| 1,5 | 1,5 | 50 |

**Patentansprüche**

1. Verbindungen der Formel I

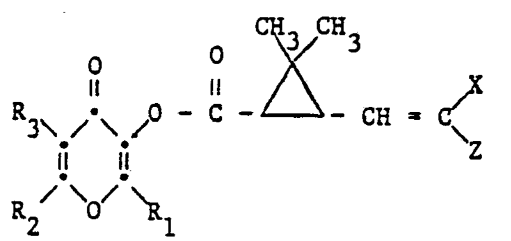

(I),

worin $R_1$, $R_2$ und $R_3$ unabhängig voneinander Wasserstoff; Halogen; $C_1$—$C_8$-Alkyl, welches unsubstituiert oder durch einen oder mehrere Substituenten aus der Gruppe Thiocyan, Hydroxy, Halogen, $C_1$—$C_4$-Alkoxy, $C_1$—$C_4$-Alkylthio, Acyloxy oder $R_4$ substituiert ist; $C_3$—$C_6$-Alkenyl, welches unsubstituiert oder durch $R_5$ substituiert ist; $C_3$—$C_6$-Alkinyl, welches unsubstituiert oder durch $R_6$ substituiert ist; oder $R_7$ bedeuten, wobei $R_4$, $R_5$, $R_6$ und $R_7$ für Phenyl, welches unsubstituiert oder durch maximal 3 gleiche oder verschiedene Substituenten aus der Gruppe Halogen, Nitro, Cyan, $C_{1-4}$-Carbonsäure, $C_{1-4}$-Carbonsäure-$C_{1-8}$-alkylester, $C_{1-8}$-Alkyl, $C_{3-6}$-Alkenyl, $C_{3-6}$-Alkinyl, Halogen-$C_{1-8}$-alkyl, $C_{1-8}$-Alkoxy oder $C_{1-8}$-Alkylthio substituiert ist; und X und Z Fluor, Chlor, Brom oder Trifluormethyl bedeuten.

2. Verbindungen der Formel I gemäss Anspruch 1, dadurch gekennzeichnet, dass $R_1$, $R_2$ und $R_3$ die im Anspruch 1 angegebenen Bedeutungen haben und X und Z für Chlor stehen.

3. Verbindungen der Formel I gemäss Anspruch 1, dadurch gekennzeichnet, dass $R_2$ die im Anspruch 1 angegebenen Bedeutungen hat, $R_1$ und $R_3$ Wasserstoff und X und Z Chlor bedeuten.

4. Verbindungen der Formel I gemäss Anspruch 1, dadurch gekennzeichnet, dass $R_1$, X und Z die im Anspruch 1 angegebenen Bedeutungen haben und $R_2$ und $R_3$ für Wasserstoff stehen.

5. Verbindungen der Formel I gemäss Anspruch 1, dadurch gekennzeichnet, dass X und Z die im Anspruch 1 angegebenen Bedeutungen haben und $R_1$ $C_1$—$C_4$-Alkyl und $R_2$ und $R_3$ Wasserstoff bedeuten.

6. 2 - (2,2 - Dichlorvinyl) - 3,3 - dimethyl - cyclopropancarbonsäure - (2 - methyl - 4 - pyron - 3 - yl) - ester.

7. 2 - (2,2 - Dichlorvinyl) - 3,3 - dimethyl - cyclopropancarbonsäure - (2 - äthyl - 4 - pyron - 3 - yl) - ester.

8. Verfahren zur Herstellung von Verbindungen der Formel I gemäss Anspruch 1, dadurch gekennzeichnet, dass man

a) eine Verbindung der Formel II

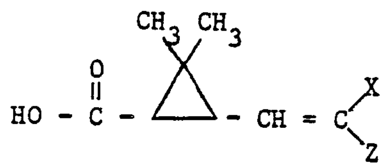 (II),

worin X und Z die für Formel I angegebenen Bedeutungen haben, mit einer Verbindung der Formel III

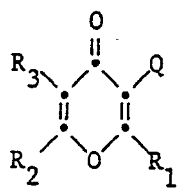 (III),

worin $R_1$, $R_2$ und $R_3$ die für Formel I angegebenen Bedeutungen haben und Q für einen nucleofugen Rest steht, umsetzt oder

b) eine Verbindung der Formel IV

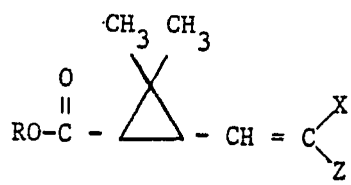 (IV),

worin X und Z die für Formel I angegebenen Bedeutungen haben und R für einen organischen Rest steht, mit einer Verbindung der Formel V

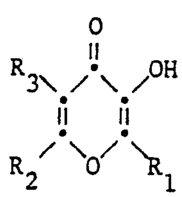 (V),

worin $R_1$, $R_2$ und $R_3$ die für Formel I angegebenen Bedeutungen haben, umsetzt oder

c) eine Verbindung der Formel VI

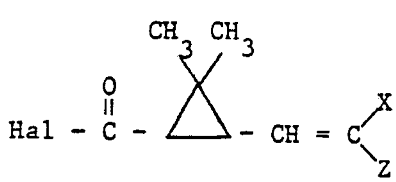 (VI),

worin X und Z die für Formel I angegebenen Bedeutungen haben und Hal für ein Halogenatom steht, mit einer Verbindung der Formel V umsetzt.

9. Mittel zum Schützen von Kulturpflanzen gegen schädigende Wirkungen von Agrarchemikalien, dadurch gekennzeichnet, dass es als mindestens eine aktive Komponente eine Verbindung der Formel I gemäss Anspruch 1 enthält.

10. Mittel nach Anspruch 9, dadurch gekennzeichnet, dass es 0,1 bis 99 Gewichtsprozent Wirkstoff der

Formel I und 1 bis 99,9 Gewichtsprozent eines festen oder flüssigen Zusatzstoffes, darunter 0 bis 25 Gewichtsprozent eines Tensides, enthält.

11. Mittel nach Anspruch 10, dadurch gekennzeichnet, dass es 0,1 bis 95 Gewichtsprozent Wirkstoff der Formel I, 5 bis 99,8 Gewichtsprozent eines festen oder flüssigen Zusatzstoffes und 0,1 bis 25 Gewichtsprozent eines Tensides enthält.

12. Mittel nach Anspruch 9, dadurch gekennzeichnet, dass es eine Verbindung der Formel I zusammen mit einem Herbizid enthält.

13. Mittel nach Anspruch 12, dadurch gekennzeichnet, dass es als Herbizid ein Chloracetanilid enthält.

14. Mittel nach Anspruch 13, dadurch gekennzeichnet, dass es als Herbizid 2 - Chlor - 2',6' - diäthyl - N - (2" - propoxyäthyl) - acetanilid enthält.

15. Mittel nach Anspruch 13, dadurch gekennzeichnet, dass es als Herbizid 2 - Chlor - 6' - äthyl - N - (2" - methoxy - 1" - methyläthyl) - acet - o - toluidid enthält.

16. Mittel nach Anspruch 12, dadurch gekennzeichnet, dass es als Herbizid 2 - [4 - (3,5 - Dichlorpyridyl - 2 - oxy) - phenoxy] - propionsäure - 2 - propinylester enthält.

17. Mittel nach Anspruch 12, dadurch gekennzeichnet, dass es als Herbizid S - Benzyl - diäthyl - thiocarbamat enthält.

18. Mittel nach Anspruch 12, dadurch gekennzeichnet, dass es als Herbizid S - 4 - Chlorbenzyl - diäthyl - thiocarbamat enthält.

19. Verfahren zum Schützen von Kulturpflanzen gegen schädigende Wirkungen von Agrarchemikalien, dadurch gekennzeichnet, dass man die Kulturpflanzen, Teile dieser Pflanzen oder für den Anbau der Kulturpflanzen bestimmte Böden mit einer Verbindung der Formel I gemäss Anspruch 1 behandelt.

20. Verfahren nach Anspruch 19, zum Schützen von Kulturpflanzen gegen schädigende Wirkungen von Pflanzenschutzmitteln.

21. Verfahren nach Anspruch 20 zum Schützen von Kulturpflanzen gegen schädigende Wirkungen von Herbiziden.

22. Verfahren nach Anspruch 21 zum Schützen von Kulturpflanzen gegen schädigende Wirkungen von Chloracetaniliden.

23. Verfahren nach Anspruch 22 zum Schützen von Kulturpflanzen gegen schädigende Wirkungen von 2 - Chlor - 2',6' - diäthyl - N - (2" - propoxyäthyl) - acetanilid.

24. Verfahren nach Anspruch 22 zum Schützen von Kulturpflanzen gegen schädigende Wirkungen von 2 - Chlor - 6' - äthyl - N - (2" - methoxy - 1" - methyläthyl) - acet - o - toluidid.

25. Verfahren nach Anspruch 21 zum Schützen von Kulturpflanzen gegen schädigende Wirkungen von 2 - [4 - (3,5 - Dichlorpyridyl - 2 - oxy) - phenoxy] - propionsäure - 2 - propinylester.

26. Verfahren nach Anspruch 21 zum Schützen von Kulturpflanzen gegen schädigende Wirkungen von S - Benzyl - diäthyl - thiocarbamat.

27. Verfahren nach Anspruch 21 zum Schützen von Kulturpflanzen gegen schädigende Wirkungen von S - 4 - Chlorbenzyl - diäthyl - thiocarbamat.

28. Verfahren nach Anspruch 21 zum Schützen von Reispflanzen.

29. Verfahren zum selektiven Bekämpfen von Unkräutern in Kulturpflanzenbeständen, dadurch gekennzeichnet, dass man die Kulturpflanzen, Teile dieser Pflanzen oder für den Anbau der Kulturpflanzen bestimmte Böden mit einem Herbizid und einer wirksamen Doses einer Verbindung der Formel I gemäss Anspruch 1 als Gegenmittel behandelt.

30. Verfahren gemäss Anspruch 29 zum selektiven Bekämpfen von Unkräutern in Kulturen von Kulturhirse, Reis, Mais, Weizen, Roggen, Gerste, Hafer, Baumwolle, Zuckerrüben, Zuckerrohr und Soja.

31. Verwendung von Verbindungen der Formel I gemäss Anspruch 1 zum Schützen von Kulturpflanzen gegen schädigende Wirkungen von Agrarchemikalien.

**Revendications**

1. Composés de formule I

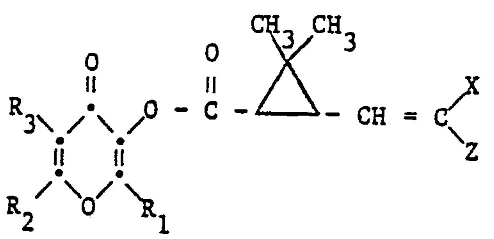

(I),

où R1, R2 et R3 représentent indépendamment l'un de l'autre un hydrogène; un halogène; un alcoyle en C1 à C8, qui est non substitué ou substitué par un ou plusieurs substituants du groupe thiocyano, hydroxy, halogène, alcoxy en C1 à C4, alcoylthio en C1 à C4, acyloxy ou R4; un alcényle en C3 à C6, qui est non substitué ou substitué par R5; un alcynyle en C3 à C6, qui est non substitué ou substitué par R6; ou R7, où R4, R5, R6 et R7 représentent un phényle, qui est non substitué ou substitué par au maximum 3 substituants identiques ou différents du groupe halogène, nitro, cyano, acide carboxylique en C1 à C4,

(alcoyl en C1 à C8) ester d'acide carboxylique en C1 à C4, alcoyle en C1 à C8, alcényle en C3 à C6, alcynyle en C3 à C6, halogénalcoyle en C1 à C8, alcoxy en C1 à C8 ou alcoylthio en C1 à C8 et X et Z représentent un fluor, un chlore, un brome ou un trifluorométhyle.

2. Composés de formule I selon la revendication 1, caractérisés en ce que R1, R2 et R3 ont les significations données dans la revendication 1 et X et Z représentent un chlore.

3. Composés de formule I selon la revendication 1, caractérisés en ce que R2 a la signification donnée dans la revendication 1, R1 et R3 représentent un hydrogène et X et Z représentent un chlore.

4. Composés de formule I selon la revendication 1, caractérisés en ce que R1, X et Z ont les significantions données dans la revendication 1 et R2 et R3 représentent un hydrogène.

5. Composés de formule I selon la revendication 1, caractérisés en ce que X et Z ont les significations données dans la revendication 1 et R1 représente un alcoyle en C1 à C4 et R2 et R3 représentent un hydrogène.

6. (2 - méthyl - 4 - pyron - 3 - yl) - ester de l'acide 2 - (2,2 - dichlorovinyl) - 3,3 - diméthyl - cyclopropanecarboxylique.

7. (2 - éthyl - 4 - pyron - 3 - yl) - ester de l'acide 2 - (2,2 - dichlorovinyl) - 3,3 - diméthyl - cyclopropanecarboxylique.

8. Procédé de préparation de composés de formule I selon la revendication 1, caractérisé en ce que

a) on fait réagir un composé de formule II

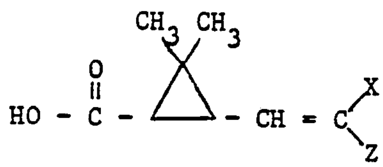 (II),

où X et Z ont les significations données pour la formule I, avec un composé de formule III

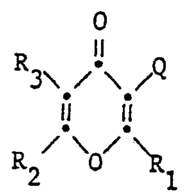 (III),

où R1, R2 et R3 ont les significations données pour la formule I et Q représente un radical nucléofuge, ou

b) on fait réagir un composé de formule IV

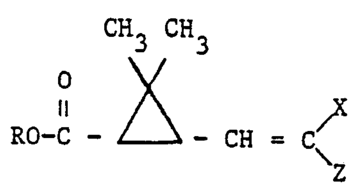 (IV),

où X et Z ont les significations données pour la formule I et R représente un radical organique, avec un composé de formule V

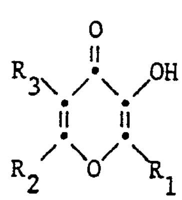 (V),

où R1, R2 et R3 ont les significations données pour la formule I, ou

c) on fait réagir un composé de formule VI

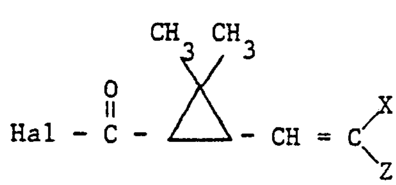 (VI),

où X et Z ont les significations données pour la formule I et Hal représente un atome d'halogène, avec un composé de formule V.

9. Agent de protection des plantes cultivées contre les effets nocifs des produits agro-chimiques, caractérisé en ce qu'il contient comme composant actif au moins un composé de formule I selon la revendication 1.

10. Agent selon la revendication 9, caractérisé en ce qu'il contient de 0,1 à 99% en poids de matière active de formule I et de 1 à 99,9% en poids d'un additif solide ou liquide, dont de 0 à 25% en poids d'un agent tensio-actif.

11. Agent selon la revendication 10, caractérisé en ce qu'il contient de 0,1 à 95% en poids de matière active de formule I, de 5 à 99,8% en poids d'un additif solide ou liquide et de 0,1 à 25% en poids d'un agent tensio-actif.

12. Agent selon la revendication 9, caractérisé en ce qu'il contient un composé de formule I avec un herbicide.

13. Agent selon la revendication 12, caractérisé en ce qu'il contient comme herbicide un chloracétanilide.

14. Agent selon la revendication 13, caractérisé en ce qu'il contient comme herbicide le 2 - chloro - 2',6' - diéthyl - N - (2" - propoxyéthyl) - acétanilide.

15. Agent selon la revendication 13, caractérisé en ce qu'il contient comme herbicide de 2 - chloro - 6' - éthyl - N - (2" - méthoxy - 1" - méthyléthyl) - acét - o - toluidide.

16. Agent selon la revendication 12, caractérisé en ce qu'il contient comme herbicide le 2-propinylester de l'acide 2 - [4 - (3,5 - dichloropyridinyl - 2 - oxy) - phénoxy] - propionique.

17. Agent selon la revendication 12, caractérisé en ce qu'il contient comme herbicide le S - benzyl - diéthyl - thiocarbamate.

18. Agent selon la revendication 12, caractérisé en ce qu'il contient comme herbicide le S - 4 - chlorobenzyl - diéthyl - thiocarbamate.

19. Procédé de protection des plantes cultivées contre les effets nocifs des produits agro-chimiques, caractérisé en ce qu'on traite les plantes cultivées, des parties de ces plantes ou des sols déterminés pour la culture des plantes cultivées avec un composé de formule I selon la revendication 1.

20. Procédé selon la revendication 19 de protection des plantes cultivées contre les effets nocifs des agents de protection des plantes.

21. Procédé selon la revendication 20 de protection des plantes cultivées contre les effets nocifs des herbicides.

22. Procédé selon la revendication 21 de protection des plantes cultivées contre les effets nocifs des chloracétanilides.

23. Procédé selon la revendication 22 de protection des plantes cultivées contre les effets nocifs du 2 - chloro - 2',6' - diéthyl - N - (2" - propoxyéthyl) - acétanilide.

24. Procédé selon la revendication 22 de protection des plantes cultivées contre les effets nocifs du 2 - chloro - 6' - éthyl - N - (2" - méthoxy - 1" - méthyléthyl) - acét - o - toluidide.

25. Procédé selon la revendication 21 de protection des plantes cultivées contre les effets nocifs du 2-propinylester de l'acide 2 - [4 - (3,5 - dichloropyridyl - 2 - oxy) - phénoxy] - propionique.

26. Procédé selon la revendication 21 de protection des plantes cultivées contre les effets nocifs du S - benzyl - diéthyl - thiocarbamate.

27. Procédé selon la revendication 21 de protection des plantes cultivées contre les effets nocifs du S - 4 - chlorobenzyl - diéthyl - thiocarbamate.

28. Procédé selon la revendication 21 de protection de plants de riz.

29. Procédé de lutte sélective contre les mauvaises herbes dans les populations de plantes cultivées, caractérisé en ce qu'on traite les plantes cultivées, des parties de ces plantes ou des sols déterminées pour la culture des plantes cultivées avec un herbicide et une dose efficace d'un composé de formule I selon la revendication 1 comme antidote.

30. Procédé selon la revendication 29 de lutte sélective contre les mauvaises herbes dans les cultures de sorgho cultivé, de riz, de maïs, de blé, de seigle, d'orge, d'avoine, de coton, de betterave sucrière, de canne à sucre et de soja.

31. Application de composés de formule I selon la revendication 1 à la protection des plantes cultivées contre les effets nocifs des produits agro-chimiques.

**Claims**

1. A compound of the formula I

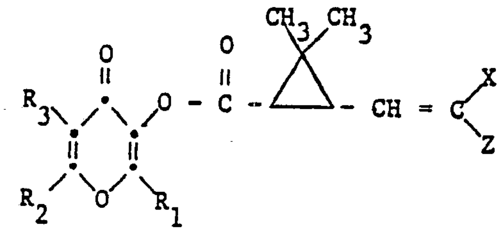

(I),

wherein $R_1$, $R_2$ and $R_3$ independently of one another are each hydrogen; halogen; $C_1$—$C_8$alkyl which is unsubstituted or substituted by one or more substituents from the group: thiocyanogen, hydroxy, halogen, $C_1$—$C_4$alkoxy, $C_1$—$C_4$alkylthio, acyloxy or $R_4$; or they are $C_3$—$C_6$alkenyl which is unsubstituted or substituted by $R_5$; or they are $C_3$—$C_6$alkynyl which is unsubstituted or substituted by $R_6$; or they are $R_7$, with $R_4$, $R_5$, $R_6$ and $R_7$ being phenyl which is unsubstituted or substituted by a maximum of three identical or different substituents from the group: halogen, nitro, cyano, $C_1$—$C_4$carboxylic acid, $C_1$—$C_4$carboxylic acid $C_1$—$C_8$alkyl ester, $C_1$—$C_8$alkyl, $C_3$—$C_6$alkenyl, $C_3$—$C_6$alkynyl, $C_1$—$C_8$haloalkyl, $C_1$—$C_8$alkoxy or $C_1$—$C_8$alkylthio; and X and Z are fluorine, chlorine, bromine or trifluoromethyl.

2. A compound of the formula I according to Claim 1, wherein $R_1$, $R_2$ and $R_3$ are as defined in Claim 1, and X and Z are chlorine.

3. A compound of the formula I according to Claim 1, wherein $R_2$ is as defined in Claim 1, $R_1$ and $R_3$ are hydrogen, and X and Z are chlorine.

4. A compound of the formula I according to Claim 1, wherein $R_1$, X and Z are as defined in Claim 1, and $R_2$ and $R_3$ are hydrogen.

5. A compound of the formula I according to Claim 1, wherein X and Z are as defined in Claim 1, and $R_1$ is $C_1$—$C_4$alkyl, and $R_2$ and $R_3$ are hydrogen.

6. 2 - (2,2 - Dichlorovinyl) - 3,3 - dimethylcyclopropanecarboxylic acid (2 - methyl - 4 - pyron - 3 - yl) ester.

7. 2 - (2,2 - Dichlorovinyl) - 3,3 - dimethylcyclopropanecarboxylic acid (2 - ethyl - 4 - pyron - 3 - yl) ester.

8. A process for producing a compound of the formula I according to Claim 1, which process comprises:

a) reacting a compound of the formula II

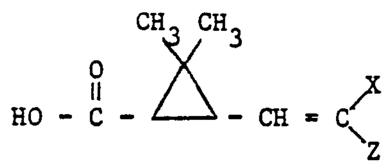

(II),

wherein X and Z are as defined under the formula I, with a compound of the formula III

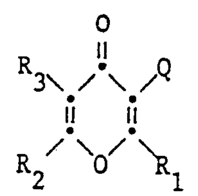

(III),

wherein $R_1$, $R_2$ and $R_3$ are as defined under the formula I, and Q is a nucleofug radical; or

b) reacting a compound of the formula IV

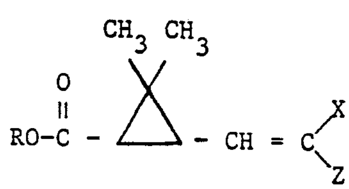

(IV),

wherein X and Z are as defined under the formula I, and R is an organic radical, with a compound of the formula V

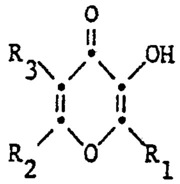

(V),

wherein $R_1$, $R_2$ and $R_3$ have the meanings defined under the formula I; or

c) reacting a compound of the formula VI

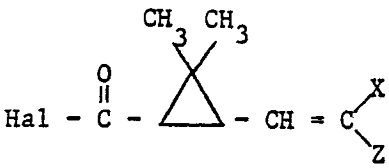

(VI),

wherein X and Z are as defined under the formula I, and Hal is a halogen atom, with a compound of the formula V.

9. A composition for the protection of cultivated plants against harmful effects of agricultural chemicals, which composition contains, as at least one active ingredient, a compound of the formula I according to Claim 1.

10. A composition according to Claim 9, which contains 0.1 to 99 per cent by weight of active ingredient of the formula I, and 1 to 99.9 per cent by weight of a solid or liquid additive, including 0 to 25 per cent by weight of a surfactant.

11. A composition according to Claim 10, which contains 0.1 to 95 per cent by weight of active ingredient of the formula I, 5 to 99.8 per cent by weight of a solid or liquid additive, and 0.1 to 25 per cent by weight of a surfactant.

12. A composition according to Claim 9, which contains a compound of the formula I together with a herbicide.

13. A composition according to Claim 12, which contains a chloroacetanilide as herbicide.

14. A composition according to Claim 13, which contains 2 - chloro - 2′,6′ - diethyl - N - (2″ - propoxyethyl)acetanilide as herbicide.

15. A composition according to Claim 13, which contains 2 - chloro - 6′ - ethyl - N - (2″ - methoxy - 1″ - methylethyl)acet - o - toluidide as herbicide.

16. A composition according to Claim 12, which contains 2 - [4 - (3,5 - dichloropyridyl - 2 - oxy)phenoxy]propionic acid 2 - propynyl ester as herbicide.

17. A composition according to Claim 12, which contains S-benzyldiethyl thiocarbamate as herbicide.

18. A composition according to Claim 12, which contains S - 4 - chlorobenzyldiethyl thiocarbamate as herbicide.

19. A process for protecting cultivated plants against harmful effects of agricultural chemicals, which process comprises treating the cultivated plants, parts of these plants, or soils intended for the growing of cultivated plants, with a compound of the formula I according to Claim 1.

20. A process according to Claim 19 for protecting cultivated plants against harmful effects of plant protection products.

21. A process according to Claim 20 for protecting cultivated plants against harmful effects of herbicides.

22. A process according to Claim 21 for protecting cultivated plants against harmful effects of chloroacetanilides.

23. A process according to Claim 22 for protecting cultivated plants against harmful effects of 2 - chloro - 2′,6′ - diethyl - N - (2″ - propoxyethyl)acetanilide.

24. A process according to Claim 22 for protecting cultivated plants against harmful effects of 2 - chloro - 6′ - ethyl - N - (2″ - methoxy - 1″ - methylethyl)acet - o - toluidide.

25. A process according to Claim 21 for protecting cultivated plants against harmful effects of 2 - [4 - (3,5 - dichloropyridyl - 2 - oxy)phenoxy]propionic acid 2-propynyl ester.

26. A process according to Claim 21 for protecting cultivated plants against harmful effects of S-benzyldiethyl thiocarbamate.

27. A process according to Claim 21 for protecting cultivated plants against harmful effects of S - 4 - chlorobenzyldiethyl thiocarbamate.

28. A process according to Claim 21 for protecting rice plants.

29. A process for selectively controlling weeds in crops of cultivated plants, which process comprises treating the cultivated plants, parts of these plants, or soils intended for growing cultivated plants, with a herbicide and an effective dose of a compound of the formula I, according to Claim 1, as antidote.

30. A process according to Claim 29 for selectively controlling weeds in crops of cultivated millet, rice, maize, wheat, rye, barley, oats, cotton, sugar beet, sugar cane and soya bean.

31. A method of protecting cultivated plants against harmful effects of agricultural chemicals, which methods comprises applying to the cultivated plants or to the locus thereof an effective amount of a compound of the formula I according to Claim 1.